# EUROPEAN PATENT APPLICATION

(11) **EP 4 763 270 A1**
(43) Date of publication of application: **24.06.2026**
(21) Application number: 24220697.7
(22) Date of filing: 17.12.2024
(51) Int. Cl.: A61N 1/04, A61N 1/20, A61N 1/32

(54) **WOUND DRESSING**

(71) Applicant: Ennocure Medtech Ltd, 3883000 Maor (IL)
(72) Inventor: Hod, Manuela, 3883000 Maor (IL); Fogel, Ofer, 3883000 Maor (IL)
(74) Representative: Schwarz & Partner Patentanwälte GmbH

(57) **Abstract**

The invention relates to a multi-layer wound dressing (1), comprising a wound-touching layer (2), at least one electric layer (3) comprising electrodes (6) and an interface (7) for individually controlling and powering the electrodes (6), wherein the electrodes (6) are configured to emit an electric signal, and an adhesive layer (4), wherein the wound-touching layer (2) and the electric layer (3) each have perforations which allow liquid to pass through, wherein the adhesive layer (4) establishes direct contact with the biological tissue, wherein the wound dressing (1) further comprises an absorbing layer (5), wherein the electric layer (3) is located between the wound-touching layer (2) and the absorbing layer (5), wherein the absorbing layer (5) is configured to absorb a liquid traveling through the perforations of the wound-touching layer (2) and the electric layer (3). The invention further relates to a method for detecting tampering in a system comprising a wound dressing and a computing device (9).

## Description

### Field of the invention

The present invention relates to wound dressings to be placed on biological tissue.

### Background of the invention

Wound dressings are an integral component of medical care, serving as a protective barrier and promoting the natural healing process of injured tissue. A wound dressing is generally understood as a material or combination of materials applied to a wound to facilitate healing, provide protection from external contaminants, manage exudate, and maintain an optimal moisture balance within the wound environment.

The benefits of using wound dressings extend beyond basic protection. They contribute significantly to wound management by controlling moisture levels, preventing infection, reducing pain, and providing mechanical protection to the tissue beneath. The prevention of desiccation (drying out) and maceration (overhydration) through appropriate moisture management can accelerate the healing process. Moreover, advanced wound dressings may incorporate antimicrobial agents, bioactive molecules, or even delivery systems for controlled-release medication, enhancing their therapeutic impact.

The process of wound healing is a complex biological process, involving hemostasis, inflammation, proliferation, and remodeling. Effective wound dressings support these stages by creating an environment conducive to cell restoration. For example, a moist environment can foster cellular activity and can accelerate the formation of new tissue. These advances not only expedite healing but also improve the functional and cosmetic outcomes of the tissue repair process.

An essential consideration in the development and selection of wound dressings is their biocompatibility and ability to integrate seamlessly with biological tissue. Dressings that can be safely applied to biological tissue without causing adverse reactions are important, as they interact directly with sensitive, damaged areas. Materials used in wound dressings must be nontoxic, non-allergenic, and biocompatible to minimize the need for frequent changes and potential complications during the healing process.

In recent years, innovative wound dressings that incorporate electrodes capable of delivering controlled electric fields have emerged as promising tools for enhancing tissue healing. These electroactive dressings, known as electroceutical dressings, use the principles of electrical stimulation to influence cellular behavior and accelerate wound closure. By generating electric fields, these dressings can enhance cell migration, which is a critical step in tissue regeneration.

For example, WO 2024121841 A1 discloses a multi-layer wound dressing, comprising electrodes which are capable of transmitting stimulating signals to the wound. A printed circuit board (PCB) layer accommodates a computing device configured to control the stimulating signals.

An objective of the invention is to provide a wound dressing which improves on the known wound patches.

### Summary of the invention

This objective is achieved by providing a wound dressing as defined in claim 1. In a further aspect of the invention, the objective is achieved by a method according to claim 12. Preferred embodiments of the invention are defined in the dependent claims, the description, and the drawings.

According to the invention, a multi-layer wound dressing to be placed on biological tissue is provided, the wound dressing comprising a wound-touching layer, at least one electric layer comprising a plurality of electrodes and an interface for individually controlling and powering the electrodes, wherein the electrodes are configured to emit an electric signal towards the biological tissue, and an adhesive layer, wherein the wound-touching layer and the electric layer each have a plurality of perforations which allow liquid to pass through, wherein the adhesive layer at least partially extends beyond the wound-touching layer and establishes direct contact with the biological tissue, wherein the wound dressing further comprises an absorbing layer, wherein the electric layer is located between the wound-touching layer and the absorbing layer, wherein the absorbing layer is configured to absorb a liquid traveling through the perforations of the wound-touching layer and the electric layer.

The wound dressing according to the invention offers several advantages over known wound dressings. The absorbing layer is beneficial for managing the moisture level of the biological tissue below the wound dressing, as wound exudate can be absorbed by the absorbing layer. By placing the electric layer between the wound-touching layer and the absorbing layer, the electric layer is still close enough to enhance the healing process by emitting electric fields. As the absorbing layer is physically separated from the biological tissue, there has to be a way for exudate to travel from the wound to the absorbing layer. This is achieved by the plurality of perforations in the electric layer, which allow a liquid to pass through.

The wound dressing according to the invention further offers the advantage of having the ability to maintain a carefully controlled microenvironment at the biological tissue. By requiring the exudate to travel through the wound dressing to reach the absorbing layer, it is possible to maintain an optimal moisture balance directly at the wound surface. This design can help keep the wound bed sufficiently moist to promote cellular activities essential for healing, while simultaneously preventing excessive moisture that could lead to maceration and impede recovery. Additionally, this separation allows the incorporation of intermediary layers that could include antimicrobial barriers or bioactive components that selectively filter and neutralize harmful pathogens, further protecting the wound and enhancing healing outcomes.

The physical nature of the biocidal effect achieved through low-intensity electric signals is effective against a wide range of pathogens and is unlikely to lead to the development of biological resistance. Low intensity electric signals disrupt biofilm adhesion and prevents their spread. When combined with antibiotics, it facilitates their penetration into the biofilm structure, thereby enhancing the effectiveness of biofilm treatment.

The low intensity signaling, applied in specific sequences, exhibits bactericidal properties by disrupting bacterial membranes. For example, the sequences can be from DC to 10 Hz with pulse widths from a few milliseconds to 1 hour with duty cycles from 10% to 90%. The voltage can be from 1 mV up to 10 V. The electric current through the biological tissue is 1 mA or less.

In another aspect of the invention, a system is provided, the system comprising a wound dressing according to the invention and a computing device comprising a battery, a memory and a computing unit, wherein the computing device is connectable to the interface of the electric layer, wherein the computing device is configured to provide the wound dressing with electric power.

The system comprising the wound dressing and the computing device offers both flexibility and health benefits. The battery, i.e. the power source for the wound dressing is housed separately within the computing unit, allowing for significant advantages in design and application. This separation alleviates the need for a compact battery within the dressing itself, facilitating greater flexibility in the positioning of both the dressing and the power source. Consequently, the wound dressing can be applied to various anatomical locations without restrictions posed by the battery size, as the computing unit, containing the larger battery, can be placed in an area that minimizes discomfort for the patient. Additionally, this computing unit is capable of both powering and precisely controlling the electrodes embedded in the wound dressing. By enabling individual electrode control, the computing device can optimize electrical stimulation in a targeted manner, thereby enhancing the healing process and promoting more efficient tissue repair.

In yet another aspect of the invention, the wound dressing can be used in a method for detecting a property of biological tissue, wherein the method comprises the following steps:
- applying the wound dressing to the biological tissue such that the wound-touching layer contacts the biological tissue,
- measuring a bioimpedance signal using at least two electrodes, wherein the electrodes are powered by the battery of the computing device, wherein the bioimpedance signal is stored in the memory of the computing device,
- detecting a wound perimeter as a first property of the biological tissue by analyzing the bioimpedance signal in the computing unit.

Understanding the perimeter of the wound is advantageous for the effective application of electric fields in promoting wound healing. If the electric field extends beyond the boundaries of the wound, the additional benefits may be minimal. Conversely, when the field is accurately confined within the wound perimeter, it can significantly enhance various aspects of the healing process, amongst others: enhancing cell mitigation, increasing collagen synthesis, stimulating angiogenesis, and reducing inflammation. The delineation of the wound perimeter is achieved through bioimpedance signal measurement, a method known for its rapidity and reliability. This approach ensures precise application of the electric field, maximizing its positive impact on tissue regeneration and overall recovery. An individual bioimpedance measurement is performed using at least two electrodes, using at least three electrodes or using at least four electrodes.

In a further aspect of the invention, a method for detecting tampering in a system comprising a wound dressing and a computing device is hereby provided, wherein the wound dressing comprises a wound-touching layer, at least one electric layer comprising a plurality of electrodes and an interface for individually controlling and powering the electrodes, wherein the electrodes are configured to emit an electric signal towards the biological tissue, and an adhesive layer, wherein the adhesive layer at least partially extends beyond the wound-touching layer and establishes direct contact with the biological tissue, wherein the computing device comprises a battery, a memory and a computing unit, wherein the computing device is connectable to the interface of the electric layer, wherein the computing device is in an energized state configured to provide the wound dressing with electric power, wherein the method comprises the following steps:
- connecting the computing device to the interface of the wound dressing,
- determining, by the computing device, whether the fuse of the wound dressing is intact or impaired,
- enabling, by the computing device, the energized state if the fuse state is intact and subsequently rendering the fuse state of the wound dressing to be impaired.

According to the invention, the method for detecting tampering only enables the computing device to power the wound dressing if the computing device recognizes an intact state of the fuse. By subsequently rendering the fuse state of the wound dressing to be impaired, a repeated use of the same wound dressing is inhibited.

Repeated use of the same wound dressing can have several deleterious effects on the wound healing process. One primary concern is the increased risk of infection. Over time, microorganisms, including pathogenic bacteria and fungi, can accumulate on the surface of the wound dressing, leading to potential contamination and colonization of the wound. This can exacerbate inflammation, impair tissue repair, and, in severe cases, cause systemic infections. Additionally, the repeated application of the same dressing can lead to the buildup of exudate, cellular debris, and other biological by-products. This accumulation can create a microenvironment that is unfavorable for healing, promoting maceration of the surrounding skin and increasing the risk of wound deterioration. Such a saturated and contaminated environment can compromise the protective barrier function of the dressing, allowing exogenous irritants or pathogens to reach the wound bed. Hence, by rendering the fuse state of a used wound dressing to be impaired, the method according to the invention only enables the computing device to power the wound dressing if the computing device recognizes an intact state of the fuse, i.e. if the wound dressing has not been used before.

In another aspect of the invention, a method for treating an injured tissue is provided, the method comprising the steps:
- providing a wound dressing according to the invention,
- applying the wound dressing to the injured tissue.

This method for treating an injured tissue provides all beneficial advantages of the wound dressing according to the invention. In summary, the wound dressing provides effective moisture management of the biological tissue, facilitating an environment conducive to rapid healing. The integration of electrodes enables the emission of targeted electric fields onto the wound, enhancing cellular activities essential for tissue repair. Additionally, the wound dressing may include antimicrobial properties or bioactive agents that further protect against infection and promote optimal healing conditions. This combination of advanced moisture regulation, electrical stimulation, and protective features positions the wound dressing as a comprehensive solution for improved wound care outcomes.

In yet another aspect of the invention, a method for providing a patient with health data is hereby provided, the method comprising the steps:
- measuring a wound parameter of an injured tissue using a system comprising a wound dressing according to an aspect of the present invention and a computing device comprising a battery, a memory and a computing unit, wherein the computing device is connectable to the interface of the electric layer, wherein the computing device is in an energized state configured to provide the wound dressing with electric power,
- storing the wound parameter in the memory,
- sending the wound parameter to an external server,
- analyzing the wound parameter on the external server and generating a health data report,
- sending the health data report to an external device of the patient.

This method automatically and continuously monitors and documents wound progress. As a result, healthcare providers may receive real-time updates on patient progress online and might also manage wound care remotely. The method can also provide predictive infection alert and allows the digitalization of assessment and documentation. A personalized preventive bio-electric therapy can be tailored to both the wound bed and patient specifics. Furthermore, AI algorithms may enable early detection of infections, while the remote care solution facilitates prompt intervention by caregivers.

### Detailed description of the invention

As used herein, the term "electric layer" comprises an electric substrate, e.g. a flexible printed circuit board (PCB), with electrodes arranged thereon and connected by conductive traces. The outermost layers of the PCB comprise electrode pads, wherein the electrode pad closest to the biological tissue is the wound-touching layer of the multi-layer wound dressing.

In a preferred embodiment, the electrodes are arranged pairwise in at least two electric layers and are configured to create an electric field both in the biological tissue and in the absorbing layer, wherein the electric field is preferably suppressed inside the electric layer. In this embodiment, the electric field extends into the absorbing layer and thereby prevents bacterial colonialization within the absorbing layer. This results in an elongated lifetime of the wound dressing.

In a preferred embodiment, the interface comprises a fuse, wherein the fuse can be in an intact state and in an impaired state. The two distinct states of the fuse allow a distinction between a used wound dressing and a wound dressing that has not been used before. By rendering the fuse into the impaired state either immediately after connecting to the interface or after a certain amount of time, it is possible to inhibit different cases of reusing the same wound dressing.

In a preferred embodiment, the fuse is a mechanical fuse, preferably a thin wire fuse or a pre-scored conductor, a thermal fuse, an electronic fuse, a chemical fuse, or a combination thereof.

A mechanical fuse, such as a thin wire fuse or a pre-scored conductor, is designed to physically interrupt an electrical circuit when current exceeds a predetermined level. This type of fuse comprises a conductive wire or a strip made of a material with a low melting point. When excessive current flows through the fuse, the resulting heat generated by electrical resistance causes the wire to melt or the pre-scored section to separate, breaking the circuit and preventing further current flow.

A thermal fuse works by breaking an electrical connection when a specific temperature threshold is surpassed. It operates based on a temperature-sensitive mechanism, e.g. by using materials that melt or change state upon reaching a critical temperature.

An electronic fuse usually comprises semiconductor technology to provide advanced circuit protection. Electronic fuses use electronic control to monitor current and voltage levels. When an overcurrent or voltage anomaly is detected, the electronic fuse rapidly disconnects the circuit. This type of fuse can be reset or re-enabled once the condition has been resolved, either manually or automatically. Thus, an electronic fuse can be rendered intact again.

A chemical fuse relies on exothermic properties of specific chemical compounds to provide circuit interruption when activated by overcurrent or thermal events. This type of fuse may use materials that decompose or react when triggered by excessive electrical or thermal energy, generating gas or mechanical force that disrupts the circuit. Chemical fuses are often designed for single-use applications where high reliability is needed. The chemical reaction provides a robust and irreversible means of breaking the circuit, ensuring a complete and effective interruption.

In a preferred embodiment, the wound dressing comprises an intermediate adhesive layer positioned between the wound-touching layer and the electric layer. This adhesive layer enhances adhesion between the wound-touching layer and the electric layer.

In a preferred embodiment, the wound-touching layer has an area of 1 cm² to 900 cm², preferably 1 cm² to 500 cm², more preferably 1 cm² to 400 cm². This area allows for an application of the wound dressing with many different types of wounds.

In a preferred embodiment, the electric layer comprises 3 to 10 000 electrodes. This embodiment relates to wound dressings with just a few, but large electrodes as well as wound dressings with many, but smaller electrodes.

A wound dressing with a few large electrodes may provide more uniform current distribution across broader areas, making it suitable for treating larger wounds where consistent coverage and electrical stimulation are needed. The larger electrodes can be easier to manufacture and apply, potentially reducing costs. Such wound dressings are generally better suited for injuries where comprehensive stimulation is required to promote uniform tissue regeneration or wound healing. Additionally, fewer electrodes mean simpler circuitry and potentially more straightforward integration into the electric layer.

A wound dressing with many small electrodes can offer precise and localized control over the electrical stimulation. This configuration allows for the adjustment of current intensity and distribution to different areas of the wound, which can be particularly beneficial for irregularly shaped wounds. This also improves the overall spatial resolution of electrical stimulation, which can stimulate cellular responses more effectively in specific zones, promoting faster and more coordinated healing in complex wound sites.

In a preferred embodiment, the area of one electrode is 10 µm² to 5 cm², preferably 25 µm² to 3 cm², more preferably 150 µm² to 2 cm².

In a preferred embodiment, the electrodes take up 1% to 80%, preferably 1% to 70%, more preferably 1% to 60%, more preferably 1% to 50%, more preferably 1% to 40%, of an area of the wound-touching layer.

In a preferred embodiment, the wound-touching layer has a rectangular, circular, elliptical, polygonal, heart- or butterfly shape. In this embodiment, the wound dressing can be chosen to cover a large variety of wound shapes. It is also possible that the wound dressing features a hole, such that the shape of a circular wound dressing is substantially ring-shaped. Of course, this also applied to rectangular, elliptical, polygonal, heart- or butterfly shapes.

In a preferred embodiment, the electrodes have a rectangular, circular, elliptical or polygonal shape. Rectangular electrodes typically create an electric field that is relatively uniform within the central region of the electrode but can exhibit stronger field concentrations near the edges due to edge effects. Circular electrodes tend to generate a more radially symmetric electric field, which distributes more evenly from the center outward. This shape reduces edge effects compared to rectangular electrodes, leading to a smoother electric field distribution. Elliptical electrodes produce an electric field that has characteristics intermediate between those of circular and rectangular electrodes. Polygonal electrodes, especially those with more complex or irregular shapes, can produce highly variable electric fields, depending on the number of sides and angles involved. Sharp corners in polygonal electrodes can concentrate the electric field at those points, creating areas of high field strength that could potentially enhance stimulation at targeted sites but also risk uneven treatment distribution.

In a preferred embodiment, the wound dressing comprises at least one further sensor selected from the group consisting of a temperature sensor, a moisture sensor, a pH-sensor, an enzyme sensor, a uric acid sensor, and an oxygen sensor.

A temperature sensor in wound dressings can monitor the local temperature of the wound site. Elevated temperature may indicate the onset of infection or inflammation, signaling a need for medical intervention. Conversely, a drop in temperature could suggest poor blood circulation or reduced metabolic activity, which may slow down the healing process.

A moisture sensor is advantageous for maintaining the optimal hydration level in a wound. The right balance of moisture is crucial for effective wound healing, as a dry wound can delay cell migration and tissue repair, while excessive moisture can lead to maceration of the surrounding skin and an increased risk of infection.

A pH sensor is valuable for assessing the acidity or alkalinity of the wound exudate. Normal wound healing environments typically exhibit a slightly acidic pH, which helps fight bacteria and promote healthy cell function. An increase in pH can indicate infection or a chronic wound state, while a drop can suggest excessive acidity that may harm tissue health.

An enzyme sensor can detect specific enzymes released by the body or pathogenic microorganisms. Certain enzymes, such as proteases, are involved in the wound healing process but can become problematic when overactive, breaking down necessary proteins and impeding tissue repair.

A uric acid sensor can be particularly beneficial in monitoring metabolic activity within the wound. Elevated uric acid levels in wound exudate may reflect inflammation or underlying metabolic disorders that could impair wound healing.

An oxygen sensor is advantageous for assessing the availability of oxygen at the wound site, as adequate oxygenation is important for cellular processes such as collagen synthesis, angiogenesis, and bacterial defense. Insufficient oxygen can lead to hypoxia, slowing down healing and potentially leading to chronic wound conditions.

In a preferred embodiment, the electric layer comprises an electric substrate, preferably a flexible printed circuit board, composed of a dielectric polymer selected from the group consisting of poly(ethylene naphthalate) (PEN), poly(ethylene terephthalate) (PET), polynorbornene (PNB), poly(cyclic olefin) (PCO), polyimide (PI), polyethersulphone (PES), polyarylate (PAR), and polycarbonate (PC)In a preferred embodiment, the electrodes are composed of a metal selected from the group consisting of copper, silver and zinc.

In a preferred embodiment, the electrodes of the electric layer comprise a coating, wherein the coating is composed of a metal, graphene or a conductive polymer.

In a preferred embodiment, the wound-touching layer is composed of a biocompatible polymeric material selected from the group consisting of polyurethane-based polymers, chitosan, carboxymethylcellulose, fibrin, collagen, and synthetic hydrogels selected from but not limited to the family of polycaprolactones, poly(vinyl alcohols), polyethylene glycol/polyethylene oxide, poly(lactic acid), poly(acrylic acid), and silicones.

In a preferred embodiment, the wound-touching layer and/or the absorbing layer comprises at least one antibiotic substance and/or metal ion, preferably silver ions. This can further elongate the lifetime of the wound dressing as bacterial colonialization is further prevented.

In a preferred embodiment, the absorbing layer is composed of a polymer and is formed as a sponge, hydrogel, gellable polymer, foam, fiber, or any combination thereof.

In a further aspect of the invention, a system is provided, comprising a wound dressing according to any one of the preceding claims and a computing device comprising a battery, a memory and a computing unit, wherein the computing device is connectable to the interface of the electric layer, wherein the computing device is configured to provide the wound dressing with electric power.

In a preferred embodiment, the interface of the wound dressing comprises a fuse and the computing device is configured to render the fuse from an intact state into an impaired state. The two distinct states of the fuse allow a distinction between a used wound dressing and a wound dressing that has not been used before. By rendering the fuse into the impaired state either immediately after connecting to the interface or after a certain amount of time, it is possible to inhibit different cases of reusing the same wound dressing.

In a further aspect of the invention, the system can be used in a method for detecting a property of biological tissue, wherein the method comprises the following steps:
- applying the wound dressing to the biological tissue such that the wound-touching layer contacts the biological tissue,
- measuring a bioimpedance signal using at least two electrodes, wherein the electrodes are powered by the battery of the computing device, wherein the bioimpedance signal is stored in the memory of the computing device,
- detecting a wound perimeter as a first property of the biological tissue by analyzing the bioimpedance signal in the computing unit. In other words, this use of the system relates to a mapping process of the wound in the biological tissue.

As used herein, the term "bioimpedance" refers to the measurement of the impedance to the flow of an electrical current through biological tissues. It is based on the principle that different types of body tissues, such as muscles, fat, and fluids, exhibit varying levels of resistance and reactance when exposed to an electrical current. Bioimpedance is measured by applying a small, safe electrical current to the body and detecting the voltage response to estimate the tissue's electrical properties.

In one example, the electrodes can be operated for 5 to 20 minutes with signal frequencies of kHz up to MHz and optimized sampling rate. For instance, the electric signals used for the bioimpedance mapping can be from 1 kHz to 1 MHz with max voltage of few volts. The electric current through the biological tissue is 1 mA or less.

This mapping process enables calibration of the electrode grid, determining the optimal activation for both, the higher accuracy bioimpedance sensing and for the later electric activation of electrodes as anodes and cathodes to deliver a wound-specific electrical therapy regimen only over the open wound.

In a preferred embodiment, the method of use further comprises the steps:
- repeatedly measuring a bioimpedance signal using at least two electrodes, wherein the bioimpedance signal is stored in the memory of the computing device together with a time stamp,
- detecting a change of the wound perimeter as a second property of the biological tissue, by comparing a first wound perimeter detected at a first time stamp with a second wound perimeter detected at a second time stamp. This method can detect tampering of the wound dressing, e.g. removing the wound dressing from the biological tissue and reapplying the same wound dressing at a different position and/or rotated by an angle around an axis perpendicular to the surface of the biological tissue.

In another aspect of the invention, a method for detecting tampering in a system comprising a wound dressing and a computing device is provided, wherein the wound dressing comprises a wound-touching layer, at least one electric layer comprising a plurality of electrodes and an interface for individually controlling and powering the electrodes, wherein the electrodes are configured to emit an electric signal towards the biological tissue, and an adhesive layer, wherein the adhesive layer at least partially extends beyond the wound-touching layer and establishes direct contact with the biological tissue, wherein the computing device comprises a battery, a memory and a computing unit, wherein the computing device is connectable to the interface of the electric layer, wherein the computing device is in an energized state configured to provide the wound dressing with electric power, wherein the method comprises the following steps:
- connecting the computing device to the interface of the wound dressing,
- determining, by the computing device, whether the fuse of the wound dressing is intact or impaired,
- enabling, by the computing device, the energized state if the fuse state is intact and subsequently rendering the fuse state of the wound dressing to be impaired.

This method for detecting tampering prevents a single re-use of the same wound dressing, as the computing device does not power the electrodes if the fuse is impaired.

In a preferred embodiment, the method for detecting tampering further comprises the following steps:
- reading, by the computing device, a unique ID of the wound dressing,
- adding, by the computing device, the unique ID to a list of allowed IDs stored within the memory if the fuse state is intact,
- enabling, by the computing device, the energized state if the fuse state is impaired and if the unique ID is contained within the list of allowed IDs.

This embodiment of the method for detecting tampering allows for an accidental unplugging of the computing device by allowing the use of a used wound dressing if the unique ID has been previously recorded to the memory.

In a preferred embodiment, the method for detecting tampering further comprises the following steps:
- setting, by the computing device, a time limit corresponding to the unique ID if the fuse state is intact,
- disabling, by the computing device, the energized state if the fuse state is impaired, the unique ID is contained within the list of allowed IDs, and the time limit has expired.

This embodiment of the method for detecting tampering prevents a repeated re-use of the same wound dressing after a certain time limit. In this embodiment, the time limit can be e.g. set to one week, such that the same wound dressing could be used for one week. After this time limit, the computing device will not power the electrodes of the wound dressing.

In a preferred embodiment of the method for detecting tampering, the energized state is enabled if the unique ID passes a security check, preferably a modulo check, a parity bit check, a pattern validation check, a cyclic redundancy check, a hashing check, or a combination thereof.

In this embodiment of the method for detecting tampering, the computing device will only power the electrodes of the wound dressing if the unique ID passes a security check. This ensures that the wound dressing can only be used if the unique ID is valid, e.g. if the wound dressing was manufactured by a certain manufacturer who knows the details of the security check.

In a preferred embodiment, after enabling the energized state, the method for detecting tampering further comprises the following step:
- repeatedly measuring a bioimpedance signal at at least two electrodes, storing the measured value of the bioimpedance signal in the memory and disabling the energized state if a later bioimpedance signal differs from an earlier bioimpedance signal.

This embodiment of the method for detecting tampering prevents a user of the wound dressing from taking off the wound dressing and/or placing it on another part of the biological tissue. In order to circumvent this, a user would have to place the wound dressing to the same part of the biological tissue, exactly as it was before, which is de facto unfeasible.

In yet another aspect of the invention, a data processing device is provided, comprising means for carrying out the steps of the method for detecting tampering in a system comprising a wound dressing and a computing device. Also, a computer program product is provided, comprising instructions which, when the program is executed by the data processing device, cause the data processing device to carry out the steps of the method. Further, a computer-readable storage medium having stored thereon the computer program product is provided.

In a further aspect of the invention, a method for treating an injured tissue is provided, comprising the steps:
- providing a wound dressing according to any one of claims 1 to 18,
- applying the wound dressing to the injured tissue.

In a preferred embodiment, the method for treating an injured tissue further comprises the steps:
- providing a computing device comprising a battery, a memory and a computing unit, wherein the computing device is connectable to the interface of the electric layer, wherein the computing device is in an energized state configured to provide the wound dressing with electric power,
- connecting the computing device to the interface of the electric layer,
- detecting an outer perimeter of the injured tissue by measuring a bioimpedance signal using at least two electrodes and saving the outer perimeter in the memory,
- energizing, by the computing device, those electrodes that are located within the outer perimeter.

This embodiment introduces low-intensity electric signaling as a strategy to treat pathogens only over open wound. The bioimpedance wound analysis (mapping and sensing) is used to generate a personalized electric signaling and allows ideal and safe conditions for treatment.

In a further aspect of the invention, a method for providing a patient with health data, comprising the steps:
- measuring a wound parameter of an injured tissue using a system comprising a wound dressing according to any one of claims 1 to 19 and a computing device comprising a battery, a memory and a computing unit, wherein the computing device is connectable to the interface of the electric layer, wherein the computing device is in an energized state configured to provide the wound dressing with electric power,
- storing the wound parameter in the memory,
- sending the wound parameter to an external server,
- analyzing the wound parameter on the external server and generating a health data report,
- sending the health data report to an external device of the patient.

The present invention is further illustrated by the following embodiments, however, without being restricted thereto:
1. A multi-layer wound dressing to be placed on biological tissue, comprising a wound-touching layer, at least one electric layer comprising a plurality of electrodes and an interface for individually controlling and powering the electrodes, wherein the electrodes are configured to emit an electric signal towards the biological tissue, and an adhesive layer, wherein the wound-touching layer and the electric layer each have a plurality of perforations which allow liquid to pass through, wherein the adhesive layer at least partially extends beyond the wound-touching layer and establishes direct contact with the biological tissue, wherein the wound dressing further comprises an absorbing layer, wherein the electric layer is located between the wound-touching layer and the absorbing layer, wherein the absorbing layer is configured to absorb a liquid traveling through the perforations of the wound-touching layer and the electric layer.
2. The wound dressing according to embodiment 1, wherein the electrodes are arranged pairwise in at least two electric layers and are configured to create an electric field both in the biological tissue and in the absorbing layer, wherein the electric field is preferably suppressed inside the electric layer.
3. The wound dressing according to any one of the preceding embodiments, wherein the interface comprises a fuse, wherein the fuse can be in an intact state and in an impaired state.
4. The wound dressing according to embodiment 3, wherein the fuse is a mechanical fuse, preferably a thin wire fuse or a pre-scored conductor, a thermal fuse, an electronic fuse, a chemical fuse, or a combination thereof.
5. The wound dressing according to any one of the preceding embodiments, wherein the wound dressing comprises an intermediate adhesive layer positioned between the wound-touching layer and the electric layer.
6. The wound dressing according to any one of the preceding embodiments, wherein the wound-touching layer has an area of 1 cm² to 900 cm², preferably 1 cm² to 500 cm², more preferably 1 cm² to 400 cm².
7. The wound dressing according to any one of the preceding embodiments, wherein the electric layer comprises 3 to 10 000 electrodes.
8. The wound dressing according to any one of the preceding embodiments, wherein the area of one electrode is 10 µm² to 5 cm², preferably 25 µm² to 3 cm², more preferably 150 µm² to 2 cm².
9. The wound dressing according to any one of the preceding embodiments, wherein the electrodes take up 1% to 80%, preferably 1% to 70%, more preferably 1% to 60%, more preferably 1% to 50%, more preferably 1% to 40%, of an area of the wound-touching layer.
10. The wound dressing according to any one of the preceding embodiments, wherein the wound-touching layer has a rectangular, circular, elliptical, polygonal, heart- or butterfly shape.
11. The wound dressing according to any one of the preceding embodiments, wherein the electrodes have a rectangular, circular, elliptical or polygonal shape.
12. The wound dressing according to any one of the preceding embodiments, wherein the wound dressing comprises at least one further sensor selected from the group consisting of a temperature sensor, a moisture sensor, a pH-sensor, an enzyme sensor, a uric acid sensor, and an oxygen sensor.
13. The wound dressing according to any one of the preceding embodiments, wherein the electric layer comprises an electric substrate, preferably a flexible printed circuit board, composed of a dielectric polymer selected from the group consisting of polyethylene naphthalate) (PEN), poly(ethylene terephthalate) (PET), polynorbornene (PNB), poly(cyclic olefin) (PCO), polyimide (PI), polyethersulphone (PES), polyarylate (PAR), and polycarbonate (PC).
14. The wound dressing according to any one of the preceding embodiments, wherein the electrodes are composed of a metal selected from the group consisting of copper, silver and zinc.
15. The wound dressing according to any one of the preceding embodiments, wherein the electrodes of the electric layer comprise a coating, wherein the coating is composed of a metal, graphene or a conductive polymer.
16. The wound dressing according to any one of the preceding embodiments, wherein the wound-touching layer is composed of a biocompatible polymeric material selected from the group consisting of polyurethane-based polymers, chitosan, carboxymethylcellulose, fibrin, collagen, and synthetic hydrogels selected from but not limited to the family of polycaprolactones, poly(vinyl alcohols), polyethylene glycol/polyethylene oxide, poly(lactic acid), poly(acrylic acid), and silicones.
17. The wound dressing according to any one of the preceding embodiments, wherein the wound-touching layer and/or the absorbing layer comprises at least one antibiotic substance and/or metal ion, preferably silver ions.
18. The wound dressing according to any one of the preceding embodiments, wherein the absorbing layer is composed of a polymer and is formed as a sponge, hydrogel, gellable polymer, foam, fiber, or any combination thereof.
19. A system comprising a wound dressing according to any one of the preceding embodiments and a computing device comprising a battery, a memory and a computing unit, wherein the computing device is connectable to the interface of the electric layer, wherein the computing device is configured to provide the wound dressing with electric power.
20. The system according to embodiment 19, wherein the interface of the wound dressing comprises a fuse and the computing device is configured to render the fuse from an intact state into an impaired state.
21. Use of the system according to any one of embodiments 19 to 20 in a method for detecting a property of biological tissue, wherein the method comprises the following steps:
   - applying the wound dressing to the biological tissue such that the wound-touching layer contacts the biological tissue,
   - measuring a bioimpedance signal using at least two electrodes, wherein the electrodes are powered by the battery of the computing device, wherein the bioimpedance signal is stored in the memory of the computing device,
   - detecting a wound perimeter as a first property of the biological tissue by analyzing the bioimpedance signal in the computing unit.
22. Use according to embodiment 21, wherein the method further comprises the steps:
   - repeatedly measuring a bioimpedance signal using at least two electrodes, wherein the bioimpedance signal is stored in the memory of the computing device together with a time stamp,
   - detecting a change of the wound perimeter as a second property of the biological tissue, by comparing a first wound perimeter detected at a first time stamp with a second wound perimeter detected at a second time stamp.
23. A method for detecting tampering in a system comprising a wound dressing and a computing device, wherein the wound dressing comprises a wound-touching layer, at least one electric layer comprising a plurality of electrodes and an interface for individually controlling and powering the electrodes, wherein the electrodes are configured to emit an electric signal towards the biological tissue, and an adhesive layer, wherein the adhesive layer at least partially extends beyond the wound-touching layer and establishes direct contact with the biological tissue, wherein the computing device comprises a battery, a memory and a computing unit, wherein the computing device is connectable to the interface of the electric layer, wherein the computing device is in an energized state configured to provide the wound dressing with electric power, wherein the method comprises the following steps:
   - connecting the computing device to the interface of the wound dressing,
   - determining, by the computing device, whether the fuse of the wound dressing is intact or impaired,
   - enabling, by the computing device, the energized state if the fuse state is intact and subsequently rendering the fuse state of the wound dressing to be impaired.
24. The method according to embodiment 23, wherein the method further comprises the following steps:
   - reading, by the computing device, a unique ID of the wound dressing,
   - adding, by the computing device, the unique ID to a list of allowed IDs stored within the memory if the fuse state is intact,
   - enabling, by the computing device, the energized state if the fuse state is impaired and if the unique ID is contained within the list of allowed IDs.
25. The method according to embodiment 24, wherein the method further comprises the following steps:
   - setting, by the computing device, a time limit corresponding to the unique ID,
   - disabling, by the computing device, the energized state if the fuse state is impaired, the unique ID is contained within the list of allowed IDs, and the time limit has expired.
26. The method according to embodiment 24 or 25, wherein the energized state is enabled if the unique ID passes a security check, preferably a modulo check, a parity bit check, a pattern validation check, a cyclic redundancy check, a hashing check, or a combination thereof.
27. The method according to any of embodiments 23 to 26, wherein after enabling the energized state, the method further comprises the following step:
   - repeatedly measuring a bioimpedance signal at at least two electrodes, storing the measured value of the bioimpedance signal in the memory and disabling the energized state if a later bioimpedance signal differs from an earlier bioimpedance signal.
28. A data processing device comprising means for carrying out the steps of the method of any of embodiments 23 to 27.
29. A computer program product comprising instructions which, when the program is executed by the data processing device of embodiment 28, cause the data processing device to carry out the steps of the method of any of embodiments 23 to 27.
30. A computer-readable storage medium having stored thereon the computer program product of embodiment 29.
31. A method for treating an injured tissue, comprising the steps:
   - providing a wound dressing according to any one of embodiments 1 to 18,
   - applying the wound dressing to the injured tissue.
32. The method according to embodiment 30, wherein the method further comprises the steps:
   - providing a computing device comprising a battery, a memory and a computing unit, wherein the computing device is connectable to the interface of the electric layer, wherein the computing device is in an energized state configured to provide the wound dressing with electric power,
   - connecting the computing device to the interface of the electric layer,
   - detecting an outer perimeter of the injured tissue by measuring a bioimpedance signal using at least two electrodes and saving the outer perimeter in the memory,
   - energizing, by the computing device, those electrodes that are located within the outer perimeter.
33. The method according to embodiment 30, wherein the method further comprises the steps:
   - providing a computing device comprising a battery, a memory and a computing unit, wherein the computing device is connectable to the interface of the electric layer, wherein the computing device is in an energized state configured to provide the wound dressing with electric power,
   - connecting the computing device to the interface of the electric layer,
   - energizing, by the computing device, the electrodes to produce hydrogen peroxide in the wound-touching layer and/or in the absorbing layer.
34. A method for providing a patient with health data, comprising the steps:
   - measuring a wound parameter of an injured tissue using a system comprising a wound dressing according to any one of embodiments 1 to 19 and a computing device comprising a battery, a memory and a computing unit, wherein the computing device is connectable to the interface of the electric layer, wherein the computing device is in an energized state configured to provide the wound dressing with electric power,
   - storing the wound parameter in the memory,
   - sending the wound parameter to an external server,
   - analyzing the wound parameter on the external server and generating a health data report,
   - sending the health data report to an external device of the patient.

### Brief description of the drawings

The present invention will now be described by way of illustrative examples with reference to the accompanying drawings, in which:
Fig. 1 shows an embodiment of a wound dressing in a sectional side view.
Fig. 2 shows another embodiment of a wound dressing in a sectional side view.
Fig. 3a shows yet another embodiment of a wound dressing in a bottom view.
Fig. 3b shows the wound dressing of Fig. 3a along the cut A-A.
Fig. 3c shows a detailed view of the wound dressing of Fig. 3b.
Fig. 4 shows another embodiment of a wound dressing in a perspective view.
Fig. 5a shows a computing device in a perspective view.
Fig. 5b shows the computing device of Fig. 5a in a top view.
Fig. 5c shows the computing device of Fig. 5a in a front view.
Fig. 5d shows the computing device of Fig. 5a in a side view.
Fig. 6 shows an embodiment of a system comprising a wound dressing and a computing device.

### Detailed description of the drawings

Reference will now be made in detail to embodiments, examples of which are illustrated in the accompanying drawings. Effects and features of the exemplary embodiments, and implementation methods thereof will be described with reference to the accompanying drawings. In the drawings, like reference numerals denote like elements, and redundant descriptions are omitted. The present invention, however, may be embodied in various different forms, and should not be construed as being limited to only the illustrated embodiments herein. Rather, these embodiments are provided as examples so that this disclosure will be thorough and complete and will fully convey the aspects and features of the present invention to those skilled in the art. Processes, elements, and techniques not considered necessary to those having ordinary skill in the art for a complete understanding of the aspects and features of the present invention may not be described. In the drawings, the relative sizes of elements, layers, and regions may be exaggerated for clarity. The below-described embodiments are merely illustrative for the principles of the present invention. It is understood that modifications and variations of the arrangements and the details described herein will be apparent to others skilled in the art. It is the intent, therefore, to be limited only by the scope of the impending patent claims and not by the specific details presented by way of description and explanation of the embodiments herein.

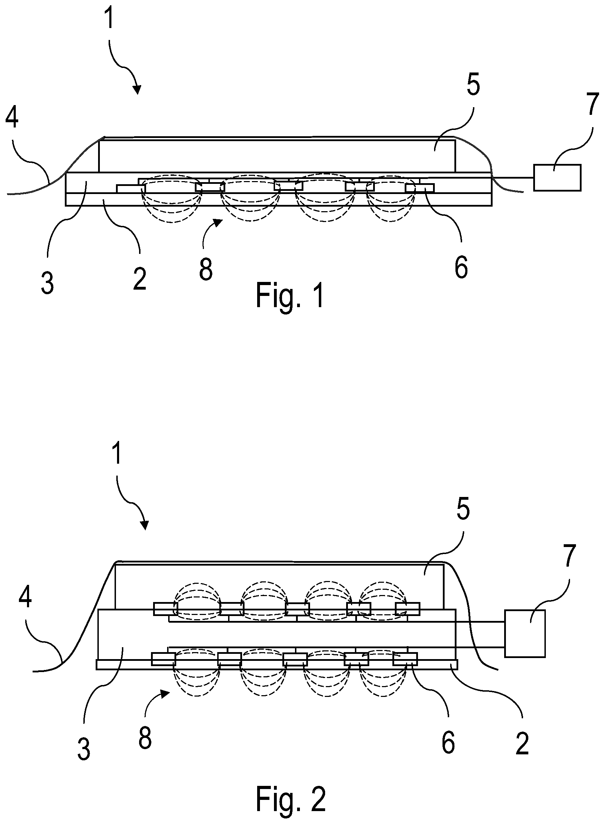

Fig. 1 shows an embodiment of a multi-layer wound dressing 1, comprising a wound-touching layer 2, an electric layer 3, an adhesive layer 4, and an absorbing layer 5, wherein the electric layer 3 is located between the wound-touching layer 2 and the absorbing layer 5. The wound dressing 1 can be applied to biological tissue, such that the wound-touching layer 2 contacts a wound of the biological tissue. The adhesive layer 4 at least partially extends beyond the wound-touching layer 2 and establishes direct contact with the biological tissue surrounding the wound.

The electric layer 3 comprises a plurality of electrodes 6 and an interface 7 for individually controlling and powering the electrodes 6, wherein the electrodes 6 are configured to emit an electric signal 8 towards the biological tissue. In the sectional view of Fig. 1, only five electrodes 6 are depicted for simplicity. The wound dressing 1 according to the invention can comprise 3 to 10 000 electrodes 6 in total.

The electric layer 3 comprises an electronic substrate, for example a flexible printed circuit board (PCB), with the electrodes 6 arranged thereon and connected by conductive traces. Another example for the electronic substrate can be a screen printed electric layer. The outermost layers of the PCB comprise electrode pads, wherein the electrode pad closest to the biological tissue is the wound-touching layer 2 of the multi-layer wound dressing 1.

The wound-touching layer 2 can have an area of 1 cm² to 900 cm², preferably 1 cm² to 500 cm², more preferably 1 cm² to 400 cm². The area of one electrode 6 can be 10 µm² to 5 cm², preferably 25 µm² to 3 cm², more preferably 150 µm² to 2 cm². Depending on the size of the wound-touching layer 2 and the electrodes 6, the electrodes 6 take up 1% to 80%, preferably 1% to 70%, more preferably 1% to 60%, more preferably 1% to 50%, more preferably 1% to 40%, of an area of the wound-touching layer 2.

The wound-touching layer 2 and the electric layer 3 each have a plurality of perforations (not shown) which allow liquid to pass through. The absorbing layer 5 is configured to absorb a liquid traveling through the perforations of the wound-touching layer 2 and the electric layer 3. This can be wound exudate, which is absorbed by the absorbing layer 5 in order to manage the moisture level of the wound.

In this embodiment, the interface 7 comprises a fuse (not shown), wherein the fuse can be in an intact state and in an impaired state. The two distinct states of the fuse allow a distinction between a used wound dressing 1 and a wound dressing 1 that has not been used before. By rendering the fuse into the impaired state either immediately after connecting to the interface 7 or after a certain amount of time, it is possible to inhibit different cases of reusing the same wound dressing 1.

The wound dressing 1 can optionally comprise an intermediate adhesive layer positioned between the wound-touching layer 2 and the electric layer 3 to enhance adhesion.

By creating an electric field, the electrodes 6 convert water present in or near the biological tissue into hydrogen peroxide.

Fig. 2 shows another embodiment of a wound dressing 1 in a sectional side view. The wound dressing 1 again comprises a wound-touching layer 2, an electric layer 3, an adhesive layer 4, and an absorbing layer 5, wherein the electric layer 3 is located between the wound-touching layer 2 and the absorbing layer 5. In this embodiment, the electrodes 6 are arranged in two layers, in particular they are arranged pairwise.

In this embodiment, the electrodes 6 are configured to create an electric field 8 both in the biological tissue and in the absorbing layer 5, wherein the electric field 8 is preferably suppressed inside the electric layer 3. In this embodiment, the electric field 8 extends into the absorbing layer 5 and thereby prevents bacterial colonialization within the absorbing layer 5. This results in an elongated lifetime of the wound dressing 1.

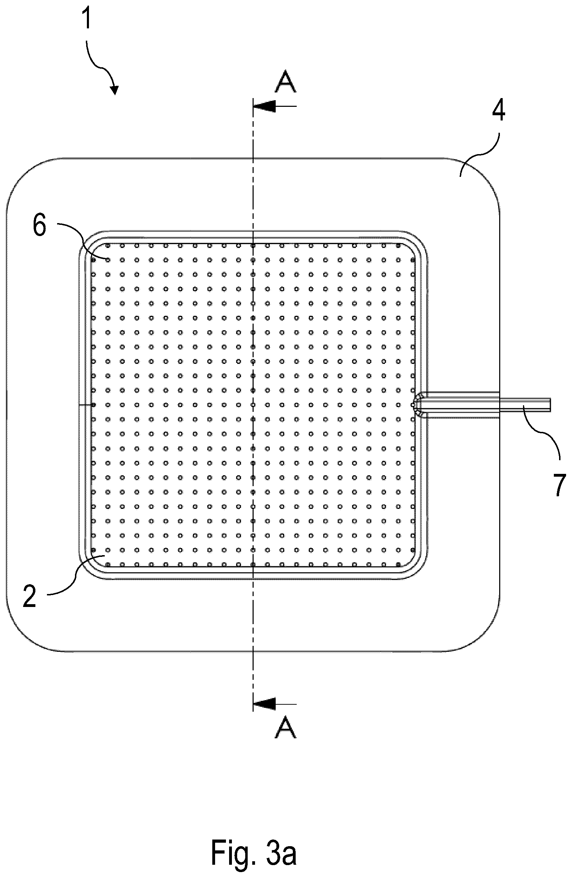

Fig. 3a shows yet another embodiment of a wound dressing 1 in a bottom view. The adhesive layer 4 extends beyond the wound-touching layer 2 along the entire perimeter of the wound-touching layer 2. In Fig. 3a, a plurality of electrodes 6 is depicted, wherein each electrode is connected to the interface 7 via conductive traces (not shown). The wound dressing 1 also comprises an electric layer 3 and an absorbing layer 5, although these layers are not visible from the bottom view.

The wound dressing 1 has a substantially rectangular shape with rounded corners. However other shapes are of course possible, e.g. circular, elliptical, polygonal, heart- or butterfly shapes.

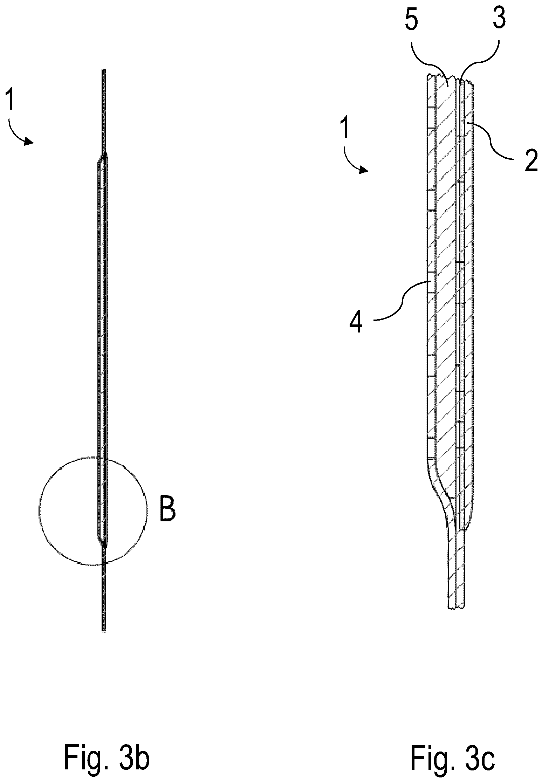

Fig. 3b shows the wound dressing 1 of Fig. 3a along the cut A-A and Fig. 3c shows a detailed view B of the wound dressing 1 of Fig. 3b.

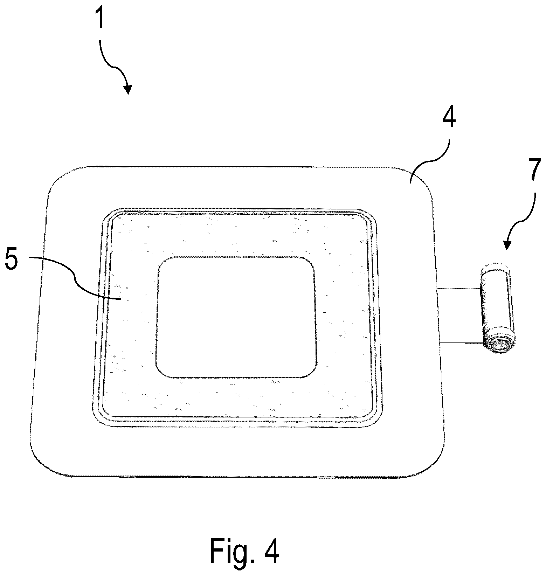

Fig. 4 shows another embodiment of a wound dressing 1 in a top perspective view. The wound dressing 1 again comprises a wound-touching layer 2, an electric layer 3, an adhesive layer 4, and an absorbing layer 5, wherein the electric layer 3 is located between the wound-touching layer 2 and the absorbing layer 5. In this embodiment, the wound dressing 1 has a hole, such that the wound-touching layer 2, the electric layer 3, the adhesive layer 4 and the absorbing layer 5 are essentially ring-shaped.

For a detailed description of the embodiment shown in Fig. 4, reference is made to the description of Figs. 1 to 3c.

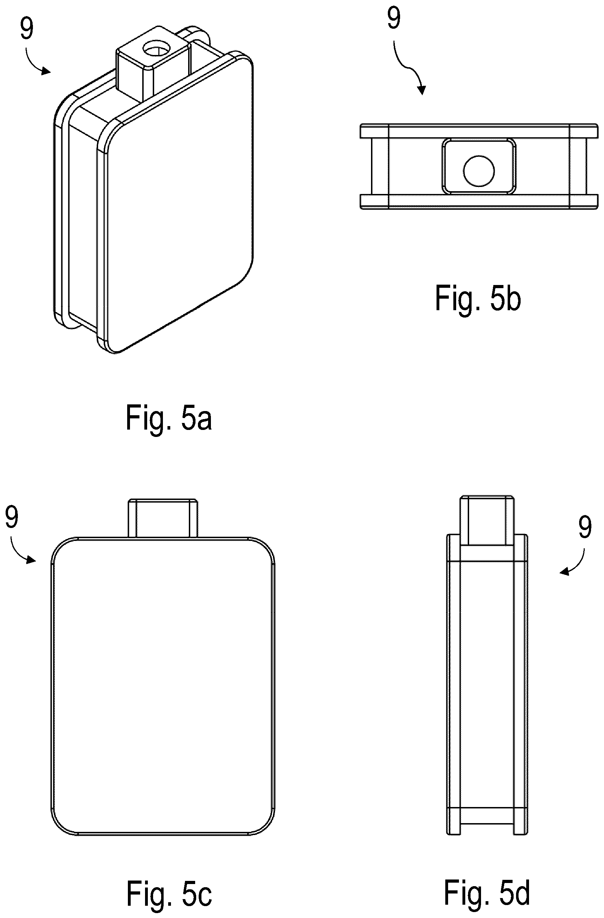

Fig. 5a shows a computing device 9 in a perspective view. The computing device 9 comprises a battery, a memory and a computing unit (each not shown) and is connectable to the interface of the electric layer 3 of a wound dressing 1. When connected, the computing device 9 is configured to provide the wound dressing 1 with electric power. In particular, the computing device 9 is configured to individually power and control the electrodes 6.

Together, the wound dressing 1 and the computing device 9 form a system that offers both flexibility and health benefits. The battery, i.e. the power source for the wound dressing is housed separately within the computing unit, allowing for significant advantages in design and application. This separation alleviates the need for a compact battery within the dressing itself, facilitating greater flexibility in the positioning of both the dressing and the power source. Consequently, the wound dressing can be applied to various anatomical locations without restrictions posed by the battery size, as the computing unit, containing the larger battery, can be placed in an area that minimizes discomfort for the patient. Additionally, this computing unit is capable of both powering and precisely controlling the electrodes embedded in the wound dressing. By enabling individual electrode control, the computing device can optimize electrical stimulation in a targeted manner, thereby enhancing the healing process and promoting more efficient tissue repair.

Fig. 5b shows the computing device of Fig. 5a in a top view, Fig. 5c shows the computing device of Fig. 5a in a front view, and Fig. 5d shows the computing device of Fig. 5a in a side view.

If the interface of the wound dressing comprises a fuse, the computing device can be configured to render the fuse from an intact state into an impaired state. This enables the system to be used in a method for detecting tampering, as will be discussed in more detail below.

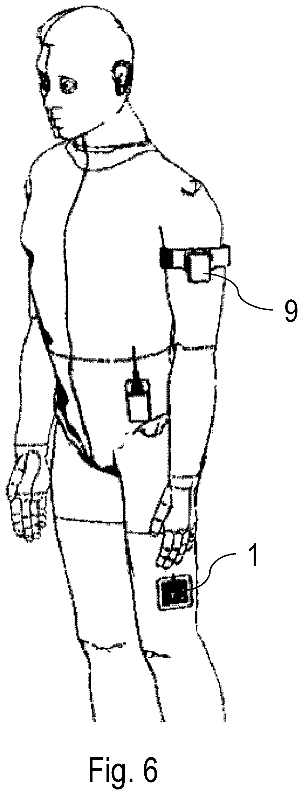

Fig. 6 shows an embodiment of a system comprising a wound dressing and a computing device 9. In use, the wound dressing 1 can be applied to any part of biological tissue. The computing device 9 is connected to the wound dressing 1, however the computing device can be positioned separately from the wound dressing 1. For example, referring to Fig. 6, the wound dressing 1 is applied to a leg of a user and the computing device 9 is located at an arm of the user.

The system of a wound dressing 1 and a computing device 9 can be used in a method for detecting a property of biological tissue. This method comprises first the step of applying the wound dressing 1 to the biological tissue such that the wound-touching layer 2 contacts the biological tissue. Next, a bioimpedance signal is measured using at least two, at least three or at least four electrodes 6, wherein the electrodes 6 are powered by the battery of the computing device 9, wherein the bioimpedance signal is stored in the memory of the computing device 9. Next, a wound perimeter can be detected as a first property of the biological tissue by analyzing the bioimpedance signal in the computing unit 9.

Understanding the perimeter of the wound is advantageous for the effective application of electric fields in promoting wound healing. If the electric field extends beyond the boundaries of the wound, the additional benefits may be minimal. Conversely, when the field is accurately confined within the wound perimeter, it can significantly enhance various aspects of the healing process, amongst others: enhancing cell mitigation, increasing collagen synthesis, stimulating angiogenesis, and reducing inflammation. The delineation of the wound perimeter is achieved through bioimpedance signal measurement, a method known for its rapidity and reliability. This approach ensures precise application of the electric field, maximizing its positive impact on tissue regeneration and overall recovery.

Optionally, the method of use can further comprise the following steps. The bioimpedance signal can be measured repeatedly using the plurality of electrodes 6, wherein the bioimpedance signal is stored in the memory of the computing device 9 together with a time stamp. As a result, a change of the wound perimeter can be detected as a second property of the biological tissue, by comparing a first wound perimeter detected at a first time stamp with a second wound perimeter detected at a second time stamp. This method can also detect tampering of the wound dressing, e.g. removing the wound dressing from the biological tissue and reapplying the same wound dressing at a different position and/or rotated by an angle around an axis perpendicular to the surface of the biological tissue.

An elaborate method for detecting tampering employs a system comprising a wound dressing and a computing device 9, wherein the wound dressing comprises a wound-touching layer 2, at least one electric layer 3 comprising a plurality of electrodes 6 and an interface 7 for individually controlling and powering the electrodes 6, wherein the electrodes 6 are configured to emit an electric signal towards the biological tissue, and an adhesive layer 4, wherein the adhesive layer 4 at least partially extends beyond the wound-touching layer 2 and establishes direct contact with the biological tissue, wherein the computing device 9 comprises a battery, a memory and a computing unit, wherein the computing device 9 is connectable to the interface 7 of the electric layer 3, wherein the computing device 9 is in an energized state configured to provide the wound dressing with electric power.

Referring to Fig. 6, the method for detecting tampering first comprises the step of connecting the computing device 9 to the interface 7 of the wound dressing. Next, the computing device 9 determines whether the fuse of the wound dressing is intact or impaired. As a result, the computing device 9 enables the energized state if the fuse state is intact and subsequently renders the fuse state of the wound dressing to be impaired. By subsequently rendering the fuse state of the wound dressing to be impaired, a repeated use of the same wound dressing is inhibited.

Repeated use of the same wound dressing can have several deleterious effects on the wound healing process. One primary concern is the increased risk of infection. Over time, microorganisms, including pathogenic bacteria and fungi, can accumulate on the surface of the wound dressing, leading to potential contamination and colonization of the wound. This can exacerbate inflammation, impair tissue repair, and, in severe cases, cause systemic infections. Additionally, the repeated application of the same dressing can lead to the buildup of exudate, cellular debris, and other biological by-products. This accumulation can create a microenvironment that is unfavorable for healing, promoting maceration of the surrounding skin and increasing the risk of wound deterioration. Such a saturated and contaminated environment can compromise the protective barrier function of the dressing, allowing exogenous irritants or pathogens to reach the wound bed. Hence, by rendering the fuse state of a used wound dressing to be impaired, the method according to the invention only enables the computing device to power the wound dressing if the computing device recognizes an intact state of the fuse, i.e. if the wound dressing has not been used before.

In short, this method prevents a single re-use of the same wound dressing, as the computing device does not power the electrodes if the fuse is impaired.

To further prevent tampering, the method can comprise the following steps. When connecting the wound dressing to the computing device 9, the computing device 9 reads a unique ID of the wound dressing. Next, the computing device 9 adds the unique ID to a list of allowed IDs stored within the memory if the fuse state is intact. Thus, the computing device registers an unused wound dressing and remembers its unique ID. As a result, the computing device 9 only enables the energized state if the fuse state is impaired and if the unique ID is contained within the list of allowed IDs.

This embodiment of the method for detecting tampering allows for an accidental unplugging of the computing device by allowing the use of a used wound dressing if the unique ID has been previously recorded to the memory.

To further prevent tampering, the method can also comprise the following steps. When the wound dressing is connected to the computing device and the fuse is intact, the computing device 9 can set a time limit corresponding to the unique ID. Subsequently, the computing device 9 can disable the energized state if the fuse state is impaired, the unique ID is contained within the list of allowed IDs, and the time limit has expired.

Hence, the method prevents a repeated re-use of the same wound dressing after a certain time limit. In this embodiment, the time limit can be e.g. set to one week, such that the same wound dressing could be used for one week. After this time limit, the computing device will not power the electrodes of the wound dressing.

As a further precaution, the energized state is only enabled if the unique ID passes a security check, preferably a modulo check, a parity bit check, a pattern validation check, a cyclic redundancy check, a hashing check, or a combination thereof.

In this embodiment of the method for detecting tampering, the computing device will only power the electrodes of the wound dressing if the unique ID passes a security check. This ensures that the wound dressing can only be used if the unique ID is valid, e.g. if the wound dressing was manufactured by a certain manufacturer who knows the details of the security check.

A modulo check is a technique used in error detection that involves dividing a set of data by a specific modulus and analyzing the remainder. This remainder acts as a verification value, ensuring that the data has not been altered during transmission or storage. The simplicity of the modulo operation allows for quick detection of certain types of errors, making it a practical tool for data integrity checks.

A parity bit check is a method used to detect errors in binary data by appending an extra bit, called the parity bit, to the data string. This bit indicates whether the total number of 'I's in the binary data is odd or even. By comparing the calculated and received parity, systems can identify single-bit errors.

A pattern validation check is employed to confirm that input data matches a specified format or structure.

A cyclic redundancy check (CRC) is a sophisticated method used to detect changes in data. This technique treats the data as a polynomial and divides it by a fixed generator polynomial, producing a remainder known as the CRC value. The CRC value is appended to the data and checked upon receipt to identify alterations.

A hashing check involves using a hash function to transform input data into a fixed-length string, known as a hash value. This process acts as a unique fingerprint for the data, allowing for verification. Even minor changes to the input will result in a substantially different hash value.

To even further prevent tampering, the method can comprise the following steps. The computing device 9 can instruct the wound dressing to repeatedly measure a bioimpedance signal at at least two electrodes 6, storing the measured value of the bioimpedance signal in the memory and disabling the energized state if a later bioimpedance signal differs from an earlier bioimpedance signal.

This embodiment of the method for detecting tampering prevents a user of the wound dressing from taking off the wound dressing and/or placing it on another part of the biological tissue. In order to circumvent this, a user would have to place the wound dressing to the same part of the biological tissue, exactly as it was before, which is de facto unfeasible.

It can be seen that various features are grouped together in a single embodiment for the purpose of streamlining the disclosure of the invention. This method of disclosure is not to be interpreted as reflecting an intention that the claimed embodiments require more features than are expressly recited in each claim. Rather, as the claims reflect, the inventive subject matter lies in less than all features of a single disclosed embodiment.

## Claims

1. A multi-layer wound dressing (1) to be placed on biological tissue, comprising a wound-touching layer (2), at least one electric layer (3) comprising a plurality of electrodes (6) and an interface (7) for individually controlling and powering the electrodes (6), wherein the electrodes (6) are configured to emit an electric signal (8) towards the biological tissue, and an adhesive layer (4), wherein the wound-touching layer (2) and the electric layer (3) each have a plurality of perforations which allow liquid to pass through, wherein the adhesive layer (4) at least partially extends beyond the wound-touching layer (2) and establishes direct contact with the biological tissue,
**characterized in that**
the wound dressing (1) further comprises an absorbing layer (5), wherein the electric layer (3) is located between the wound-touching layer (2) and the absorbing layer (5), wherein the absorbing layer (5) is configured to absorb a liquid traveling through the perforations of the wound-touching layer (2) and the electric layer (3).

2. The wound dressing according to claim 1, wherein the electrodes (6) are arranged pairwise in at least two electric layers (3) and are configured to create an electric field (8) both in the biological tissue and in the absorbing layer (5), wherein the electric field (8) is preferably suppressed inside the electric layer.

3. The wound dressing according to any one of the preceding claims, wherein the interface (7) comprises a fuse, wherein the fuse can be in an intact state and in an impaired state.

4. The wound dressing according to any one of the preceding claims, wherein the wound dressing (1) comprises an intermediate adhesive layer positioned between the wound-touching layer (2) and the electric layer (3).

5. The wound dressing according to any one of the preceding claims, wherein the electrodes (6) take up 1% to 80%, preferably 1% to 70%, more preferably 1% to 60%, more preferably 1% to 50%, more preferably 1% to 40%, of an area of the wound-touching layer (2).

6. The wound dressing according to any one of the preceding claims, wherein the wound dressing comprises at least one further sensor selected from the group consisting of a temperature sensor, a moisture sensor, a pH-sensor, an enzyme sensor, a uric acid sensor, and an oxygen sensor.

7. The wound dressing according to any one of the preceding claims, wherein the wound-touching layer and/or the absorbing layer comprises at least one antibiotic substance and/or metal ion, preferably silver ions.

8. The wound dressing according to any one of the preceding claims, wherein the absorbing layer (5) is composed of a polymer and is formed as a sponge, hydrogel, gellable polymer, foam, fiber, or any combination thereof.

9. A system comprising a wound dressing (1) according to any one of the preceding claims and a computing device (9) comprising a battery, a memory and a computing unit, wherein the computing device (9) is connectable to the interface (7) of the electric layer (3), wherein the computing device (9) is configured to provide the wound dressing (1) with electric power.

10. The system according to claim 9, wherein the interface (7) of the wound dressing (1) comprises a fuse and the computing device (9) is configured to render the fuse from an intact state into an impaired state.

11. Use of the system according to any one of claims 9 to 10 in a method for detecting a property of biological tissue, wherein the method comprises the following steps:
- applying the wound dressing (1) to the biological tissue such that the wound-touching layer (2) contacts the biological tissue,
- measuring a bioimpedance signal using at least two electrodes (6), wherein the electrodes (6) are powered by the battery of the computing device (9), wherein the bioimpedance signal is stored in the memory of the computing device (9),
- detecting a wound perimeter as a first property of the biological tissue by analyzing the bioimpedance signal in the computing unit.

12. A method for detecting tampering in a system comprising a wound dressing and a computing device (9), wherein the wound dressing comprises a wound-touching layer (2), at least one electric layer (3) comprising a plurality of electrodes (6) and an interface (7) for individually controlling and powering the electrodes (6), wherein the electrodes (6) are configured to emit an electric signal (8) towards the biological tissue, and an adhesive layer (4), wherein the adhesive layer (4) at least partially extends beyond the wound-touching layer (2) and establishes direct contact with the biological tissue, wherein the computing device (9) comprises a battery, a memory and a computing unit, wherein the computing device (9) is connectable to the interface (7) of the electric layer (3), wherein the computing device (9) is in an energized state configured to provide the wound dressing with electric power, wherein the method comprises the following steps:
- connecting the computing device (9) to the interface (7) of the wound dressing,
- determining, by the computing device (9), whether the fuse of the wound dressing is intact or impaired,
- enabling, by the computing device (9), the energized state if the fuse state is intact and subsequently rendering the fuse state of the wound dressing to be impaired.

13. The method according to claim 12, wherein the method further comprises the following steps:
- reading, by the computing device (9), a unique ID of the wound dressing,
- adding, by the computing device (9), the unique ID to a list of allowed IDs stored within the memory if the fuse state is intact,
- enabling, by the computing device (9), the energized state if the fuse state is impaired and if the unique ID is contained within the list of allowed IDs.

14. The method according to claim 13, wherein the method further comprises the following steps:
- setting, by the computing device (9), a time limit corresponding to the unique ID if the fuse state is intact,
- disabling, by the computing device (9), the energized state if the fuse state is impaired, the unique ID is contained within the list of allowed IDs, and the time limit has expired.

15. The method according to any of claims 12 to 14, wherein after enabling the energized state, the method further comprises the following step:
- repeatedly measuring a bioimpedance signal at at least two electrodes (6), storing the measured value of the bioimpedance signal in the memory and disabling the energized state if a later bioimpedance signal differs from an earlier bioimpedance signal.
